# EUROPEAN PATENT APPLICATION

(11) **EP 1 621 221 A1**
(43) Date of publication of application: **01.02.2006**
(21) Application number: 04722423.3
(22) Date of filing: 22.03.2004
(51) Int. Cl.: A61M 5/158

(54) **MEDICAL NEEDLE DEVICE HAVING SHIELD WITH WINGS**

(30) Priority: 04.04.2003 JP 2003101153
(71) Applicant: JMS Co. Ltd., Hiroshima-shi, Hiroshima 730-8652 (JP)
(72) Inventor: MORIWAKI, Kuniharu,, . (JP); HONGO, Susumu,, . (JP); KIYONO, Takafumi,, . (JP)
(74) Representative: Grape & Schwarzensteiner
(86) International application number: PCT/JP2004/003836
(87) International publication number: WO 2004/089449

(57) **Abstract**

The medical needle device includes a winged shield (4) that has a substantially cylindrical shield tube (4a) and a pair of wings (5, 6) positioned at a front end side of the shield tube, a hub (2) that is inserted into an inner bore of the shield tube so as to be movable in an axial direction, and a needle (1) that is mounted to a front end of the hub. An infusion tube (3) can be attached to a rear end of the hub, and a tip of the needle can be stored in the inner bore of the shield tube. The shield tube is bendable at least in a part along the axial direction when the needle protrudes from a front end of the shield tube and is latched to the shield tube. Thereby, the medical needle device is bendable in a curve in a position that is sufficiently close to the needle, while having the winged shield.

## Description

### TECHNICAL FIELD

The present invention relates to a medical needle device, and more particularly relates to a medical needle device with a winged shield for preventing needle-stick injuries, in which a needle can be stored safely after use.

### BACKGROUND ART

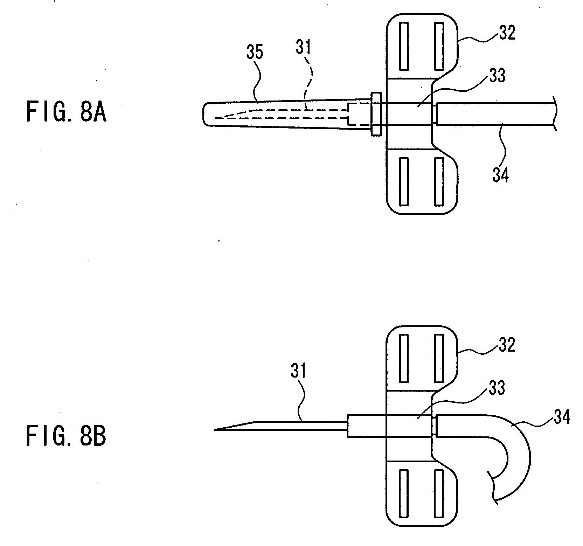

Winged medical needle devices are used widely for procedures such as infusion, blood transfusion, extracorporeal blood circulation and the like. As an example thereof, a winged indwelling needle shown in FIG. 8A and 8B is known. This winged indwelling needle has a configuration where a needle 31 is held at a front end of a hub 33 that has wings 32, and an infusion tube 34 can be attached to a rear end of the hub 33. Reference numeral 35 denotes a needle cap mounted to the needle 31. During infusion, the wings 32 are fastened onto the patient's arm or the like by adhesion tapes or the like so as to retain the insertion of the needle 31.

Meanwhile, contamination and infection due to needle-stick injuries from injection needles, insertion needles and the like have been a problem in medical centers. In particular, recently, since hepatitis B, hepatitis C, HIV (human immunodeficiency virus) and the like have become a widespread social issue, there is a demand for the means that actively prevent the occurrence of unexpected accidents such as needle-stick injuries and the like. For preventing needle-stick injuries, various kinds of injection needle devices are known to have a configuration where a cylindrical shield can slide with respect to an injection needle. That is, by sliding the cylindrical shield, the injection needle can either be exposed or stored in the shield, and when the injection needle and the insertion needle are disposed after use, each of them can be slid into the shield so as to be stored therein.

Configurations of a winged injection needle device to which the structure for preventing needle-stick injury is applied are described in, for example, JP H06(1994)-7861B, JP H05(1993)-300942A, U.S. Patent No. 4,170,933 and the like. Such a winged injection needle device is provided with wings on an outer surface of the slidable cylindrical shield, and the wings can slide together with the shield outside the injection needle. After use of the injection needle, the shield can be slid so as to cover the tip of the injection needle for preventing needle-stick injuries.

While indwelling the winged indwelling needle, wings 32 are fastened as mentioned above, and at the same time, a tube 34 may be flexed and curved (bent). For example, in the case of a general winged indwelling needle, it is, in most cases, temporarily fastened onto the patient's skin by adhesive tapes, in the state that an excess part of the tube is wound at rearward of the wings.

On the other hand, in the case of the above-mentioned conventional medical needle device with the winged shield having a function of preventing the needle-stick injuries, the probability of the bend of the shield part is not taken into consideration. For the function of preventing the needle-stick injuries, it is suitable for the shield part to be rigid, and generally impossible to bend the shield part. Therefore, as mentioned above, a part that is bendable in an inserting state is only a tube part on a rear side of the winged shield, and inevitably is located at a rear end of the needle device. However, in the light of usability at the treatment, it is preferable that the winged shield is bendable in the vicinity of the wings.

### DISCLOSURE OF THE INVENTION

It is an object of the present invention is to provide a medical needle device with a winged shield that is bendable in a position that is sufficiently close to a needle.

The medical needle device of the present invention comprises: a winged shield that has a substantially cylindrical shield tube and a pair of wings positioned at a front end side of the shield tube; a hub that is inserted into an inner bore of the shield tube so as to be movable in an axial direction; and a needle that is mounted to a front end of the hub, wherein an infusion tube can be attached to a rear end of the hub and a tip of the needle can be stored in the inner bore of the shield tube. The shield tube is bendable at least in a part in an axial direction when the needle protrudes from the front end of the shield tube and is latched to the shield tube.

### BRIEF DESCRIPTION OF DRAWINGS

FIG. 1 is a plan view showing a function of the medical needle device with a winged shield according to Embodiment 1.
FIG. 2 is a plan cross-sectional view of the medical needle device according to Embodiment 1.
FIG. 3 is a plan cross-sectional view showing another state of the medical needle device according to Embodiment 1.
FIG. 4 is a plan cross-sectional view of the medical needle device with a winged shield according to Embodiment 2.
FIG. 5 is a plan cross-sectional view showing another state of the same medical needle device.
FIG. 6 is a plan view showing a function of the same medical needle device.
FIG. 7 is a plan view of the medical needle device according to Embodiment 3.
FIGs. 8A and 8B are plan views showing a function of the medical needle device with a winged shield according to the conventional example.

### BEST MODE FOR CARRYING OUT THE INVENTION

The medical needle device of the present invention has a configuration where a hub holding a needle is mounted to an inner bore of a winged shield, and a part of a shield tube is bendable together with the hub when the needle protrudes from a front end of the shield tube. Thereby, in a inserting state, the winged shield is bendable in a position that is sufficiently close to the needle, and thus other needle devices easily can insert in proper positions.

In the medical needle device of the present invention, at least a part of the hub is made of a material having flexibility. Alternatively, the hub may have a configuration where a length of the hub is set so that, when the needle protrudes from the front end of the shield tube and is latched to the shield tube, the rear end of the hub may be positioned on a side closer to the front end of the shield tube than a rear end of the shield tube.

The shield tube may be made of a material having flexibility.

So as to allow the medical needle device to be bendable as mentioned above, it may have a configuration where the shield tube includes an extendable portion that is structured to be extendable and contractible, the needle can be moved in the axial direction of the shield tube by extending and contracting the extendable portion, and the shield tube and the hub are bendable at the extendable portion. The extendable portion preferably has a plasticity-processed accordion-like structure.

It is preferable that, when the shield tube and the hub in the inner bore of the shield tube are bent together, a minimum radius of curvature at a bent part can be 3 mm or smaller.

Embodiments of the present invention will be described below with reference to the drawings.

### (Embodiment 1)

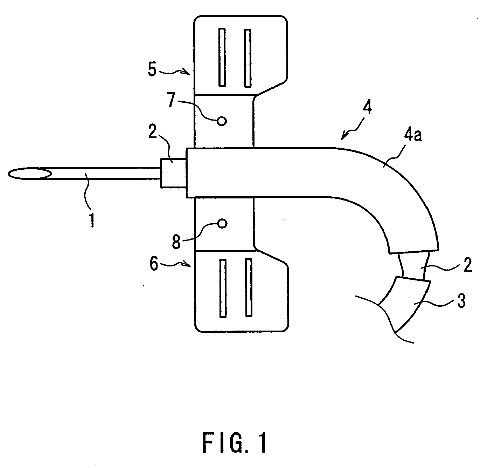

FIG. 1 is a plan view of the medical needle device according to Embodiment 1 of the present invention. Reference numeral 1 denotes a needle that is fixed to a front end of a hub 2 made of resin. To a rear end of the hub 2, a tube 3 is connected. Reference numeral 4 denotes a winged shield, which includes a substantially cylindrical shaped shield tube 4a made of resin and left and right wings 5 and 6. In the inner bore of the shield tube 4a, the needle 1 and the hub 2 are inserted, which are movable in an axial direction. The left and right wings 5 and 6 are provided at a front end portion of the shield tube 4a, that is, at the end from which the needle 1 protrudes. Here, the wings are not necessarily positioned at an edge of the front end of the shield tube 4a, but may be positioned on a side closer to the front end of the shield tube 4a than the center of the shield tube 4a as appropriate. The wings 5 and 6 are respectively jointed to both sides of the outer surface of the shield tube 4a, being symmetrical with each other about the axis of the shield tube 4a. The shield tube 4a and the hub 2 are made of flexible resin materials.

In the state shown by FIG. 1, the needle 1 protrudes from the shield tube 4a, being insertable, and the shield tube 4a and the hub 2 are bent in a curve. Thus, the shield tube 4a and the hub 2 are bendable together at least in part along the axial direction. In order to allow the shield tube 4a and the hub 2 to be bendable, they are formed to have sufficient flexibility. It is preferable that a bendable portion of the hub 2 is not folded acutely but is bendable in a curve, so as to secure a smooth flow of a drug solution. According to this configuration, the medical needle device is bendable in a position that is sufficiently close to the needle 1. Flexibility of the shield tube 4a and the hub 2 may be adjusted according to a degree of bend that is required for an embodiment of the use, but generally can satisfy the requirement for the practical use if a minimum radius of curvature at the bendable portion can be 3 mm or smaller.

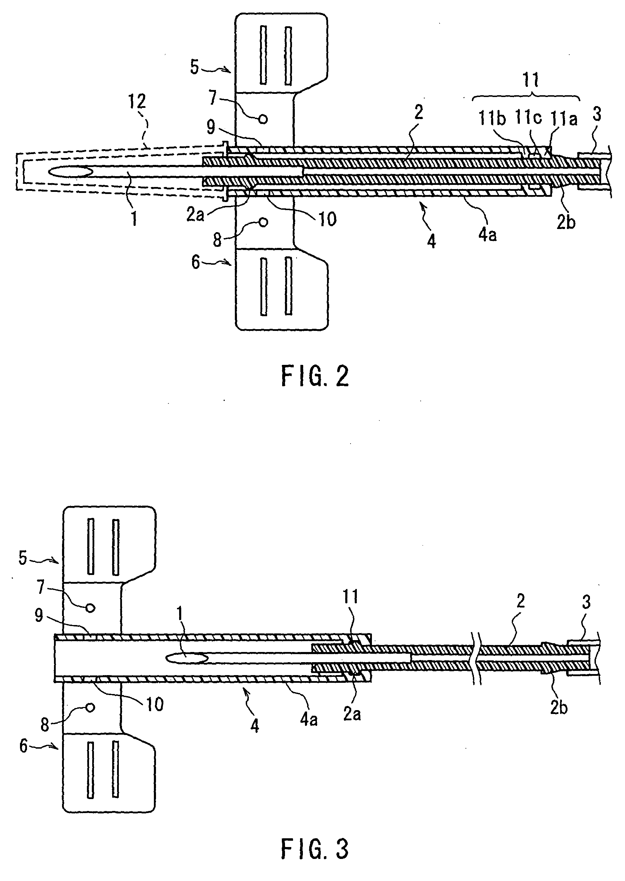

FIG. 2 shows a cross-section of the configuration of the medical needle device in the axial direction. Through holes 9 and 10 respectively are formed on right and left sides of a side wall of the shield tube 4a at the front end thereof. Wing projections 7 and 8 corresponding respectively to the through holes 9 and 10 are formed on the wings 5 and 6. In addition, a rear end latch portion 11 is formed on a rear end inner surface of the shield tube 4a. The rear end latch portion 11 includes a small diameter portion 11a and an inward annular protrusion 11b. The inward annular protrusion 11b is disposed at a predetermined interval with respect to the small diameter portion 11a, thereby forming an annular groove 11c.

The hub 2 includes a holding portion 2a formed at a front end portion thereof, and a stopper portion 2b formed at a rear end portion thereof. An outer diameter of the hub 2 is the same as, or slightly larger than inner diameters of the small diameter portion 11a and the inward annular protrusion 11b of the shield tube 4a. The outer diameter of the hub 2 may be slightly smaller than them according to an embodiment. An outer diameter of the stopper portion 2b of the hub 2 is larger than the inner diameter of the small diameter portion 11a of the shield tube 4a. Therefore, by contact of a step portion formed by the stopper portion 2b with the small diameter portion 11a of the shield tube 4a, movement of the hub 2 toward the front end of the shield tube 4a is limited. The diameter of the holding portion 2a of the hub 2 is larger than the inner diameter of the inward annular protrusion 11b of the shield tube 4a. The needle 1 can be covered by a needle cap 12 mounted to the front end of the hub 2.

When mounting a winged shield 4 to the hub 2, the hub 2 is inserted from the front end of the shield tube 4a and is moved toward a base end side. In this case, the stopper portion 2b first comes in contact with the rear end latch portion 11. Since a taper is provided to a rear end side of the stopper portion 2b as shown in the figure, the hub 2 easily can pass through the rear end latch portion 11 due to the flexibility of the resin. As a result, the medical needle device is in the state shown by FIG. 2. The hub 2 in FIG. 2 is in a position of use, while the needle 1 protrudes from the front end of the shield tube 4a by a predetermined length. In this state, as mentioned above, further movement of the needle 1 toward the front end in the axial direction is prevented by engagement of the stopper portion 2b with the small diameter portion 11a.

Usually, a medical needle device is used in the state shown by FIG. 2. An insertion operation is performed while the wings 5 and 6 are held by hand. When holding the wings 5 and 6 by hand by lifting and superposing them upwards along the outer surface of the shield tube 4a, the wing protrusions 7 and 8 respectively are inserted into the through holes 9 and 10, pass through the wall of the shield tube 4,a, and protrude into the inner bore. As a result, front ends of the wing protrusions 7 and 8 contact with the step portion formed at a rear side of the holding portion 2a of the hub 2. Thereby, the hub 2 is prevented from moving rearward, and the needle 1 can be held by the winged shield 4. During this action, by pressing the both wings 5 and 6 so as to sandwich them with fingers, the needle 1 can be held by a force that is sufficient for the insertion action.

When disposing of the medical needle device after use, in order to prevent needle-stick injuries, a tip of the needle 1 is stored in the shield tube 4a as shown in FIG. 3. When the hub 2 is retracted from the position shown in FIG. 2 toward the rear end of the shield tube 4a, the holding portion 2a firstly comes in contact with the inward annular protrusion 11b. Since the holding portion 2a has the taper on the rear side thereof, the holding portion 2a easily can pass through the inward annular protrusion 11b by further moving the hub 2 forcibly. When the holding portion 2a passes through the inward annular protrusion 11b, the holding portion 2a is engaged with the annular groove 11c as shown in FIG. 3. Due to this engagement, the movement of the hub 2 in the shield tube 4a in the axial direction is prevented, thereby maintaining the state that the needle 1 is stored in the shield tube 4a. The above-mentioned holding configuration is one of the examples, and another configuration may be applied for holding the needle 1 in the shield tube 4a.

### (Embodiment 2)

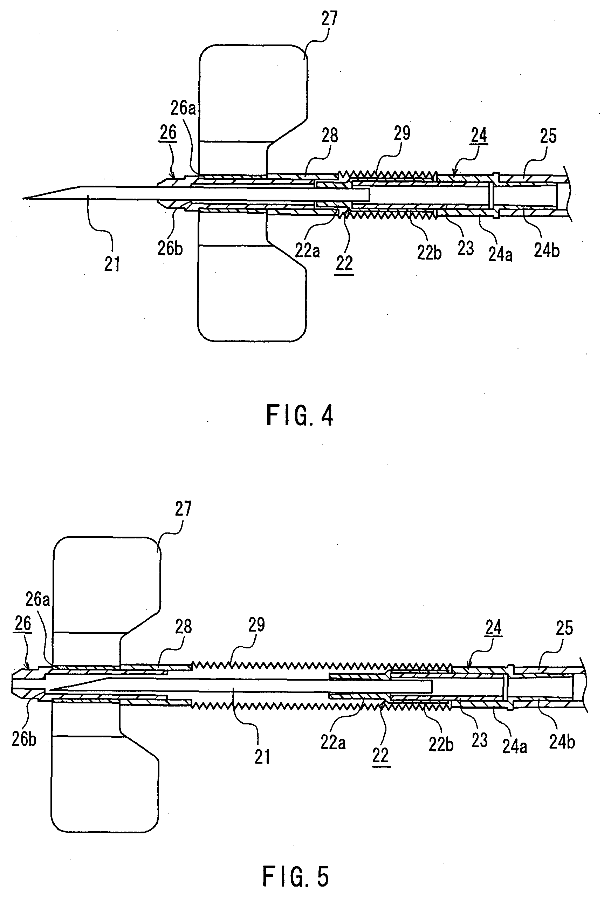

The medical needle device with a winged shield according to Embodiment 2 is shown in FIG. 4. A needle 21 is held in an inner bore of a cylindrical hub 22. The hub 22 includes a small diameter portion 22a on a needle 21 side and a large diameter portion 22b on a rear side of the small diameter portion 22a. The needle 21 is held by fitting to the small diameter portion 22a. One of the end portions of a connecting tubule 23 is fitted to the large diameter portion 22b. The other end portion of the connecting tubule 23 is fitted to a large diameter portion 24a of a cylindrical splicer 24. Therefore, the medical needle device has a configuration where the hub 22 and the splicer 24 are connected via the connecting tubule 23. A tube 25 is provided by fitting to a small diameter portion 24b of the splicer 24. As mentioned above, a continuous hole is formed throughout from the tube 25 to the needle 21. The hub 22 is made of a flexible resin material.

Reference numeral 26 denotes a cylindrical front-side shield tube, and the needle 21 can move in an inner bore of the front-side shield tube 26. Wings 27 are mounted to the front-side shield tube 26 at a cylindrical portion at the pivot of the wings 27, and are rotatable around the front-side shield tube 26. Movement of the wings 27 toward the front end of the needle 21 is inhibited by an outer-surface step portion 26a provided on an outer surface of the front-side shield tube 26. Moreover, movement of the wings 27 toward a base end is prevented by a wing stopper 28 fixed on a rear side of the front-side shield tube 26.

Reference numeral 29 denotes an extendable portion that is made of polyethylene and has an accordion-like structure, one end of the extendable portion 29 being fixed so as to fit on an outer surface of the wing stopper 28, and the other end of the extendable portion 29 being fixed so as to fit on an outer surface of a large diameter portion 24a of the splicer 24. A shield tube is composed of the extendable portion 29 and the front-side shield tube 26. Due to extension and contraction of the extendable portion 29, the front-side shield tube 26 can move outside the needle 21. Thereby, the needle 21 either can be covered by and stored in the front-side shield 26, or can be exposed. In addition, the accordion-like structure is plasticity-processed so that it can maintain its state without external force (in a free state) after being extended or contracted by the external force. Therefore, the extendable portion 29 can maintain its extended or contracted state with a required length within an extendable and contractible range. As a result, an exposure length of the needle 21 that is exposed from the front-side shield tube 26 can be adjusted freely within a certain range.

The front-side shield tube 26 has an inner diameter that is small at a front end thereof and is slightly larger on a side closer to a rear end thereof than the front end, and includes an inner-surface step portion 26b.

FIG. 5 shows a state that the needle 21 is stored in the front-side shield tube 26 by extending the extendable portion 29 of the injection needle device with the above-mentioned configuration. In this state, the needle 21 is restrained from protruding from the front-side shield tube 26. That is, a tip of the needle 21 is in contact with the inner-surface step portion 26b of the front-side shield tube 26, thereby being prevented from protrusion. In order to allow the needle 21 to protrude, it is necessary to adjust a position of the tip of the needle 21 to be in a hole at the front end of the front-side shield tube 26, so that the possibility of the unexpected protrusion is accordingly small.

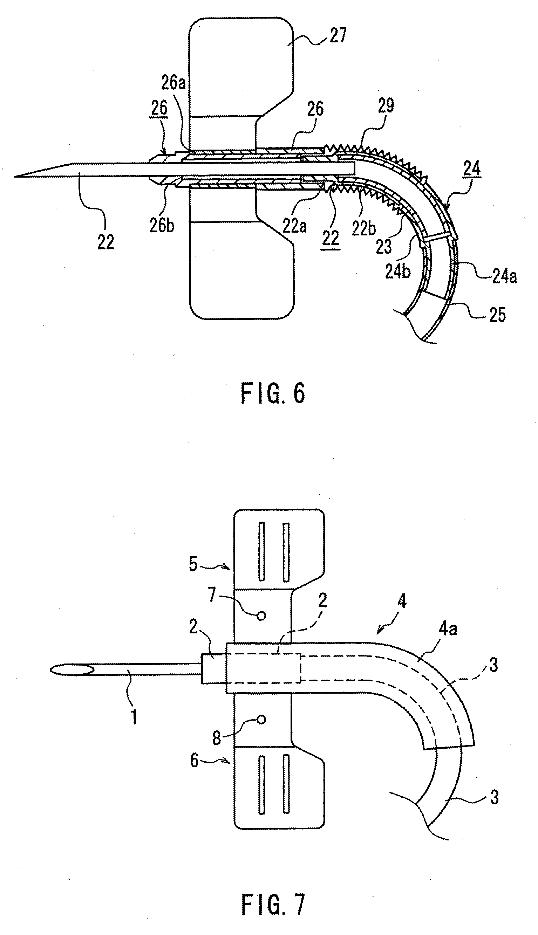

FIG. 6 shows the medical needle device according to the present embodiment when being bent at the extendable portion 29. The extendable portion 29 is bendable as shown in the figure. Flexibility of the hub 22 is set so that it can be bent at least at the extendable portion 29. In this state, the needle 21 protrudes from the front-side shield tube 26, thus being in an insertable state. Therefore, the medical needle device is bendable in a position that is sufficiently close to the needle 21.

For the extendable portion 29, a material that can extend and contract plastically may be used, and examples thereof include materials that are used for straws or the like, which can maintain either an extended state or contracted state. Specifically, polyolefin such as polyethylene and polypropylene, polyvinyl chloride resin, styrenic thermoplastic elastomer and the like are preferable. The material of the protector is not particularly limited as long as it is used for needle bases or wings of conventional medical needle devices. In addition, the front-side shield tube 26 and the wings 27 may be made of different materials, which may be preferable in terms of usability or functionality. This is because, the wings 27 generally are required to be flexible so as to conform with the patient's skin, on the other hand, the front-side shield tube 26 is required to be rigid so as to hold and store the needle 21.

In the present embodiment, it is preferable that, when the extendable portion 29 and the hub 22 in the inner bore of the extendable portion 29 are bent together, a minimum radius of curvature at the bent part can be 3 mm or smaller.

### (Embodiment 3)

FIG. 7 shows the medical needle device with a winged shield according to Embodiment 3. The present embodiment is a modified example of Embodiment 1. In the present embodiment, the length of the hub 2 is shorter than that in Embodiment 1. That is, the length of the hub 2 is set so that, when the needle 1 protrudes from the front end of the shield tube 4a and is latched to the shield tube 4a, the rear end of the hub 2 may be positioned on a side closer to the front end of the shield tube 4a than the rear end of the shield tube 4a. In this state, the hub 2 is positioned corresponding to only a part of the front end of the shield tube 4a in the axial direction, and only the tube 3 corresponds to the rear side portion of the shield tube 4a. Thus, when the shield tube 4a is bent, the tube 3 is bent accordingly, and thus can be wound easily. The length of the hub 2 is preferably set so that, when the needle 1 protrudes from the front end of the shield tube 4a and is latched to the shield tube 4a, the rear end of the hub 2 may be positioned on a side closer to the front end of the shield tube 4a than the center of the shield tube 4a in the axial direction.

The configuration of the present embodiment is also applicable in the case where the connecting tubule 23 shown in FIG. 6, instead of the tube 3, is connected to the rear side of the hub 2.

### INDUSTRIAL APPLICABILITY

The medical needle device of the present invention, which has a winged shield, is bendable in a curve in a position that is sufficiently close to a needle, thus easily can be in a state for adapting to an embodiment of use.

## Claims

1. A medical needle device with winged shield, comprising:
a winged shield that has a substantially cylindrical shield tube and a pair of wings positioned at a front end side of the shield tube;
a hub that is inserted into an inner bore of the shield tube so as to be movable in an axial direction; and
a needle that is mounted to a front end of the hub,
a rear end of the hub being capable of being connected with an infusion tube and a tip of the needle being capable of being stored in the inner bore of the shield tube,
wherein the shield tube is bendable at least in a part along an axial direction when the needle protrudes from the front end of the shield tube and is latched to the shield tube.

2. The medical needle device according to claim 1, wherein at least a part of the hub is made of a material having flexibility.

3. The medical needle device according to claim 1, wherein a length of the hub is set so that, when the needle protrudes from the front end of the shield tube and is latched to the shield tube, the rear end of the hub is positioned on a side closer to the front end of the shield tube than a rear end of the shield tube.

4. The medical needle device according to any one of claims 1 to 3, wherein the shield tube is made of a material having flexibility.

5. The medical needle device according to any one of claims 1 to 3, wherein the shield tube includes an extendable portion that is structured to be extendable and contractible, the needle can be moved in the axial direction of the shield tube by extending and contracting the extendable portion, and the shield tube and the hub are bendable at the extendable portion.

6. The medical needle device according to claim 5, wherein the extendable portion has a plasticity-processed accordion-like structure.

7. The medical needle device according to claim 1, wherein, when the shield tube and the hub in the inner bore of the shield tube are bent together, a minimum radius of curvature at a bent part can be 3 mm or smaller.
